# EUROPEAN PATENT SPECIFICATION

(11) **EP 2 654 714 B1**
(45) Date of publication and mention of the grant of the patent: **25.07.2018**
(21) Application number: 11806039.1
(22) Date of filing: 09.12.2011
(51) Int. Cl.: A61K 31/351, A61K 31/435, A61K 9/00

(54) **A drug eluting patch for the treatment of localized tissue disease or defect**
Medikamentenbeschichtetes Pflaster zur Behandlung von lokalisierten Gewebeerkrankungen oder Gewebedefekten
Timbre à élution de médicament pour le traitement d'une maladie tissulaire localisée ou d'un défaut de tissu localisé

(30) Priority: 20.12.2010 US 201061425172 P
(43) Date of publication of application: 30.10.2013
(73) Proprietor: Cormatrix Cardiovascular, Inc., Roswell, GA 30076 (US)
(72) Inventor: MATHENY, Robert G., Norcross, GA 30092 (US)
(74) Representative: Müller Fottner Steinecke
(86) International application number: PCT/US2011/064115
(87) International publication number: WO 2012/087606

(56) References cited:
- WO-A2-02/067895
- US-A1- 2009 196 910
- US-A1- 2009 263 453
- US-A1- 2010 239 632
- KUMAGAI K ET AL: "THE HMG-COA REDUCTASE INHIBITOR ATORVASTATIN PREVENTS ATRIAL FIBRILLATION BY INHIBITING INFLAMMATION IN A CANINE STERILE PERICARDITIS MODEL", CARDIOVASCULAR RESEARCH - NONGWEIHUI YUYE TEKAN, NONGYEHUI * YUYESHU, TW, vol. 62, no. 1, 1 April 2004 (2004-04-01), pages 105-111, XP008036414, ISSN: 0008-6363, DOI: 10.1016/J.CARDIORES.2004.01.018

## Description

### FIELD OF THE INVENTION

The present invention is generally in the field of drug delivery to the heart, particularly for treatment of atrial fibrillation.

### BACKGROUND OF THE INVENTION

An arrhythmia is a disorder with the speed or rhythm of the heartbeat. Atrial fibrillation (AF) is the most common type of arrhythmia. The cause is a disorder in the heart's electrical system. Atrial fibrillation (AF) is a significant complication that occurs in about 30% of the patients undergoing cardiac surgery. The risk of AF increases with age. Patients over 30 years of age exhibit a 75% increase in AF for every decade of life. The molecular mechanism of acute AF following cardiac surgery is not well understood. Impaired hemodynamics, endothelial dysfunction, inflammation, oxidative stress, and thrombosis have been implicated as important contributors to AF. In AF patients, turbulent blood flow impacts nitric oxide (NO) bioavailability, producing vascular dysfunctions and activation of coagulation system which may contribute to the high rate of embolic events.

Drugs that reduce vascular inflammation and improve vascular dysfunction and NO bioavailability may be efficacious for the prevention and treatment AF. One such class of drugs that exhibit these vascular and cardiac pleotropic effects are the HMG-COA inhibitors statins. It is widely documented that statins reduce vascular inflammation, oxidative stress and improve NO generation. Statins also modulate small GTPase including Rac1 by isoprenylation. Rac1 binds to p67phox and leads to activation of the NADPH oxidase system and subsequent generation of reactive oxygen species (ROS). Indeed, Rac1 activity is closely related to ROS production and ROS generated by NADPH oxidase in response to growth factors and inflammatory cytokines is mediated by Rac1 . Importantly, statins inhibit Rac1-mediated NADPH oxidase activity and thereby reduce angiotensin II-induced ROS production and hypertrophy in smooth muscle and heart. Rac 1 has been shown to play role in the pathogenesis of AF. Transgenic mice overexpressing Rac1 exhibit conduction abnormalities and atrial fibrosis.

Observational and prospective studies have suggested beneficial effect of statin on AF (Liakopoulos OJ et al. Euro. Heart J. 29; 1548-1559 (2008)) However, results of other prospective studies have been negative. For example, Collard, et al. (J. Thorac. Cardiovasc. Surg. 2006, 132:392) and Thielman, et al. (J. Thorac. Cardiovas. Surg. 2007; 134, 1143) failed to show decrease in all cause mortality in patients using statin before cardiac surgery.

There have been a limited number of trials on the effect of systemically administered statin on atrial fibrillation. The results are inconsistent and, in most cases, not statistically significant. For example, Negi, et al., J. Cardiovasc. Electrophysiol. Apr;22(4):414-9 (2011) reported on a randomized, double-blinded, placebo-controlled trial, where patients with atrial fibrillation/flutter (AF) were randomized to receive either atorvastatin 80 mg (n = 33) or placebo (n = 31) before CV. Treatment was continued for 12 months or until AF recurred. They concluded that high-dose atorvastatin did not reduce the recurrence of AF after CV. It reduced selective markers of inflammation without affecting systemic oxidative stress. Failure of atorvastatin to prevent AF recurrence may be due to its failure to affect oxidative stress. Another study has shown that atorvastatin but not simvastatin was effective in prevention of AF (Naji et al Int. Heart J. 50, 153-160 (2009)). The inconsistent effect of statins could also be due to inadequate and inconsistent concentration at the target site when administered orally. Further limitations to the efficacy may be due to variability of patient's response to liver metabolism when delivered orally. The high dose of statins also increase the risk of liver and muscle toxicities. Alves, et al. Arg. Bras Cardiol. 2010 Sep 24. pii: S0066-782X2010005000129. [Epub ahead of print] reported on a study to evaluate whether the chronic and regular use of statins, for a period of six months, prevents atrial fibrillation after elective cardiac surgery. In the postoperative period, atrial fibrillation was present in 42 patients (39%) of the sample, including 11 (26%) people that had used statins on a regular basis in the preoperative period and 31 (74%) who had not. They concluded that the regular use of a statin, for six months or more in the preoperative period, reduced the incidence of AF after elective cardiac surgery. In a third report, Kinoshita, et al., Circ. J., vol. 74(9), pp.1846-51 (2010), reported on a study to assess the preventive effect of preoperative statin treatment on atrial fibrillation (AF) after elective isolated off-pump coronary artery bypass grafting (off-pump CABG) in propensity score-matched Japanese patients. 584 patients were retrospectively reviewed from among 770 consecutive patients undergoing isolated CABG by the same surgeon (99.2% with off-pump technique without conversion to cardiopulmonary bypass) between 2002 and 2009, after excluding emergency (n=150), chronic AF (n=30), and use of cardiopulmonary bypass (n=6). 364 patients received statin at least 5 days before operation and 220 patients received no statin. The concluded that preoperative statin significantly reduces the incidence of AF after elective isolated off-pump CABG in Japanese patients.

It is therefore an object of the present invention to provide a means for treating or preventing atrial fibrillation.

US 2010/239632 relates to treatments of pain and/or inflammation by the administration of a biodegradable drug depot film, patch, strip or sponge being implantable at or near a cardiac tissue or within a nasal or sinus cavity. US 2009/263453 relates to methods and compositions for reducing, treating or preventing pain and/or inflammation, comprising administering a therapeutically effective amount of a statin or pharmaceutically acceptable salt thereof to a target tissue site beneath the skin. US 2009/196910 relates to a preparation of statins for prolonged release. WO 02/067895 relates to implanted, sustained release dosage forms, devices and methods for the delivery of a cholesterol lowering agent. Kumagai et al. in "The HMG-CoA reductase inhibitor atorvastatin prevents atrial fibrillation by inhibiting inflammation in a canine sterile pericarditis model", Cardiovasc Res. 2004 Apr 1;62(1):105-11 relates to the evaluation of the effect of statin on AF in a canine sterile pericarditis model.

### SUMMARY OF THE INVENTION

A polymeric matrix comprising an HMG CoA reductase inhibitor, such as a statin, to cardiac tissue for use in the treatment of atrial fibrillation has been developed. In the preferred embodiment, a statin is delivered by means of a patch sutured to cardiac tissue at the time of cardiothoracic surgery.

In the most preferred embodiment, the patch is a biodegradable material providing controlled or sustained release over a prolonged period of time, such as a week. Suitable materials include extracellular matrix, or other biodegradable hydrogels or polymeric materials providing sustained or controlled release of statin at the site of application.

### BRIEF DESCRIPTION OF THE DRAWINGS

Figure 1 is a graph of cumulative release of cerivastatin from the ECM patch over time in days.
Figure 2 is a graph of relative MCP-1 mRNA levels as a function of the amount of cerivastatin (µM) *in vitro.*
Figure 3 is a graph of MCP-1 relative mRNA as a function of LPS, LPS + patch, LPS + cerivastatin + patch on day 1, LPS + cerivastatin + patch on day 2, LPS + cerivastatin + patch on day 3, and LPS + cerivastatin + patch on day 4 *in vitro.*
Figure 4 is graph of the cumulative proportion of no atrial fibrillation as a function of days after surgery, comparing placebo and atrovastatin.

### DETAILED DESCRIPTION OF THE INVENTION

### I. Compositions

### A. HMG CoA Reductase Inhibitors

Statins or HMG-CoA reductase inhibitors are a class of drug used to lower cholesterol levels by inhibiting the enzyme HMG-CoA reductase, which plays a central role in the production of cholesterol in the liver. Increased cholesterol levels have been associated with cardiovascular diseases (CVD), and statins are therefore used in the prevention of these diseases. The best known of the statins is atorvastatin, marketed as LIPITOR® and manufactured by Pfizer. As of 2010, a number of statins are on the market: atorvastatin (LIPITOR® and TORVAST®), fluvastatin (LESCOL®), lovastatin (MEVACOR®, ALTOCOR®, ALTOPREV®), pitavastatin (LIVALO®, PITAVA®), pravastatin (PRAVACHOL®, SELEKTINE®, LIPOSTAT®), rosuvastatin (CRESTOR®) and simvastatin (ZOCOR®, LIPEX®). Several combination preparations of a statin and another agent, such as ezetimibe/simvastatin, sold as VYTORIN®, are also available.

The most preferred statin is cerivastatin, (3R,5S,6E)-7-[4-(4-fluorophenyl)-5-(methoxymethyl)-2,6-bis(propan-2-yl)pyridin-3-yl]-3,5-dihydroxyhept-6-enoic acid.

Systemic dosages for statins are generally between 10-80 mg daily, with lower dosages for local delivery in a matrix being effective for treatment of a disorder such as atrial fibrillation. Various doses of cerivastatin, as desired for efficacy could be incorporated into the patch and then released over time. For example, the patch can be incubated with 10mg to achieve high concentration at the local site or 10 µg cerivastatin to achieve low concentration or any concentrations in between. The duration of application or statin release can be from one day to 30 days for prevention or treatment of AF.

### B. Matrices for Administration of HMG CoA Reductase Inhibitor

In the preferred embodiment, the inhibitors are administered from FDA approved cardiac patches formed from extracellular matrix material ("ECM") which are used for pericardial reconstruction and prevention of adhesion. These patches are used to locally deliver drugs following cardiac surgery. Pharmacologically levels of statin can be incorporated in the ECM patch and then released over time *in vivo.* Implantable acellular xenograft derived from the extracellular matrix (ECM) is used for pericardial repair and cardiac reconstruction[Badylak et al., 2003; Badylak et al., 2006; Gerdisch et al., 2009; Robinson et al., 2005] and has been proven safe and FDA approved for use in humans (Badylak, et al. 2009. Acta Biomater 5(1): 1-13.

Other natural materials may be used for drug delivery. For example, Miyagi, et al., Biomaterials 2010 Oct 28, reported on the use of a biodegradable collagen patch for delivery of VEGF for myocardial repair. Alginate scaffolds are described by Shachar, et al., in Acta Biomater. 7(1):152-62 (2011). Decellularized matrices are described by Singelyn, et al. in J Cardiovasc Transl Res. 3(5):478-86 (2010). Polymeric woven or non-woven matrices can be utilized. For example, non-biodegradable polyurethane matrices having seeded therein myoblasts is reported by Giraud, et al., in Artif Organs. 34(6):E184-92 (2010). Ito, et al., reported on the use of a polytetrafluorethylene (PTEF) patch in Ann Thorac Surg. 89(5):1620-4 (2010). Fibrinogen matrices or TachoSil, a sponge impregnated with human fibrinogen and thrombin, can be used for short term delivery, although the delivery time is preferably longer than the two to three days obtainable at most with a fibrin matrix.

Preferably the polymeric materials are biodegradable. Representative examples include the polyhydroxy acids poly(lactic acid), poly(glycolic acid) and copolymers thereof, and polyhydroxyalkanoates such as poly(4-hydroxybutyrate), all of which are FDA approved for use in humans. Poly(4-hydroxybutyrate) is available from Tepha Inc of MA and has been used in studies of heart valve leaflets made from woven meshes. A bilayered patch made of a poly[ethylene glycol]-based matrix and poly[lactide-co-caprolactone] backing layer loaded with amiodarone (10 mg per patch) is reported by Bolderman in J Thorac Cardiovasc Surg. 140(4):904-10 (2010). Chen, et al., describes an elastomeric patch derived from poly(glycerol sebacate) for delivery of cardiomyocytes differentiated from embryonic stem cells to the heart in Biomaterials. 31(14):3885 (2010).

### C. Other Bioactive Agents

Other bioactive materials can also be delivered with this technology. For example, vascular epithelial growth factor, as reported by Miyagi, et al. (2010). Other materials may be cellular, preferably autologous, such as stem cells, myoblasts or B cells. Stein, et al., reported on the reduction of fibrosis-related arrhythmias by chronic renin-angiotensin-aldosterone system inhibitors in Am J Physiol Heart Circ Physiol. 299(2):H310-21 (2010). The authors tested eplerenone; losartan; and cotreatment with eplerenone and losartan. Myocardial fibrosis increases arrhythmia vulnerability of the diseased heart. Bolderman (2010) describes administration of amiodarone.

Preferred actives to include with matrix include antiinflammatory corticosteroids, antioxidants such as liopcyanin, SiRNA for Rac1, antithrombotic and antiplatelet drugs such as Plavix.

### II. Methods of Administration

### A. Disorders to be Treated

Postoperative atrial fibrillation is the most common arrhythmic complication following cardiac surgery with a reported incidence between 31.9% and 63.6% in patients undergoing various cardiac procedures [Auer et al., 2005; Creswell et al., 1993; Echahidi et al., 2008]. Patients with postoperative AF are at significantly increased risk of hypotension, congestive heart failure, thrombo-embolic events such as a cerebrovascular accident and the need for postoperative implantation of a permanent pacemaker or implantable defibrillator. Complications of postoperative AF has been associated with an increased length of stay (LOS) in the ICU or postoperative telemetry floor. A recent study from the Commonwealth of Virginia found that the average additive cost of isolated postoperative AF following isolated CABG was $2,574 per patient [Speir et al., 2009]. Annual costs to the U.S. healthcare system due to postoperative AF and its sequelae are estimated to run in excess of $2 billion [Echahidi et al., 2008]. Further, chronic AF affects more than 3 million people in US and about 5 million in western Europe The etiology of underlying pathogenesis of chronic AF is similar which includes, inflammation, oxidative stress and fibrosis. With increase in aging population, the estimated number of cases of AF in the USA are expected to reach 15 million by 2050.

An optimum therapy for postoperative AF is yet to be available. Current interventions include beta-adrenergic blockers, perioperative amiodarone, sotalol, nondihydropyridine calcium channel blockers, magnesium sulfate and biatrial pacing F. The efficacy of these treatments is variable and accompanied with risk.

In the preferred embodiment, the composition is applied to an area having ischemic damage or atrial fibrillation. Statins should reduce the incidence of postoperative AF. The beneficial effect of statins on the pathophysiology of AF is supported by several observational and prospective studies, suggesting that statin treatment is beneficial to patients undergoing electrical cardioversion, postoperative AF, paroxymal AF, and AF associated with coronary artery disease and left ventricular dysfunction. A small prospective, randomized, placebo controlled, double blind ARMYDA-3, Atorvastatin for Reduction of Myocardial Dysrhythmia after cardiac surgery, study has shown that treatment with 40mg/day atorvastatin produced 61% reduction in risk of postoperative AF. Another recent study by Qiang et al, Circ J 73: 2244 - 2249 (2009), showed that atorvastatin 20 mg/day, initiated 1 week before elective off-pump CABG and continued in the postoperative period, significantly decreases postoperative AF.

At present, suitable pharmacological agents for the prevention of postoperative AF are not available. Several prospective studies strongly suggest that statin class of drugs reduced post-operative AF. A high dose of statin and preoperative dosing appears to be required for an optimum efficacy. Since high doses of statins are required to achieve improvement of vascular functions, the therapeutic doses of statins either do not achieve direct benefit on vascular functions or achieve only partial benefit. Further limitations to efficacy may be due to patient variability and variable drug levels at the local site. High doses of statins required to achieve blood levels that produce direct beneficial effects on vascular functions increase risk of liver and muscle toxicities.

Therefore, local delivery of statins following cardiac surgery achieves greater efficacy on vascular and cardiac functions, while avoiding the increased risk of liver and muscle toxicity. Since AF is a regional pathogenesis, and consistent levels of drug are required for optimum therapy, local delivery of statin produces maximum efficacy and minimum adverse effects.

### B. Methods of Administration

In the preferred embodiment, the drug is mixed with the material forming the matrix and the matrix formed then administered. This is the preferred method for materials such as extracellular matrix material matrices and polymeric meshes, which are sutured to the tissue to be treated at the time of implantation, typically during surgery. Alternatively, for example, in the case of a fibrinogen matrix, the drug is mixed with the fibrinogen and thrombin added, and the polymerizing mixture applied to the heart tissue.

The actual dose of statin, preferably cerivastatin, as desired for effiacy, to be incorporated into the patch for release over time can be determined by extrapolation from *in vitro* and animal studies. For example, the patch can be loaded with 10 mg/cm2 to achieve high concentrations at the local site in heart or vasculature or as low as 10 µg/cm² cerivatatin to achieve low concentrations or any concentrations in between. The duration of treatment or statin release may vary from 1 day to 30 days for the prevention of AF.

The present invention will be further understood by reference to the following non-limiting inventions.

### Example 1: In Vitro Efficacy of ECM Patch loaded with Cerivastatin

A tissue engineered ECM patch with desired cerivastatin release kinetics was prepared. Preliminary studies have demonstrated that cerivastatin can be incorporated in the ECM patch. Studies of loading conditions and release kinetics have shown that cerivastatin release kinetics can be successfully optimized to achieve drug levels that produce antiinflammatory effects. Studies were then conducted to determine optimal conditions to produce uniformly drug loaded patches for *in vivo* studies.

### Materials and Methods

### Tissue engineered pericardial repair patch preparation:

### Determination of the kinetics of cerivastatin release from the ECM

**patch:** Loading of cerivastatin in the patch was performed by incubation in different concentrations of cerivastain in phosphate buffered saline (PBS) at 25°C for 24 hours. The patch was then washed with fresh PBS three times. The kinetics of cerivastatin release was then determined by incubation in 0.5 ml of PBS at 25 °C. The supernatants were collected and replenished with fresh PBS at 4 hours, 1, 2, 3, 4, 5, 6, 7, 10, 15 days. The quantity of cerivastatin in the supernatants was determined using HPLC. All experiments were performed in triplicate.

**Determination of the cerivastatin by HPLC:** Cerivastatin was measured by separating on a C18 ultra aqueous analytical column (250mmx4.6mm, 5µm) from RESTEK. Mobile phase was mixture of 90% acetonitrile and 10% water with flow rate at 1mL/min. Cerivastatin was detected spectrophotometrically at 220 nm.

**Determination of anti-inflammatory activity of cerivastatin released from the patch:** THP-1 cells were seeded at 6×10⁵ cells/ml into 12-well transwell plates. Cells were pre-treated with cerivastatin, patch alone or cerivastatin loaded patches for 1h and then treated with 200ng/mL LPS for 24h. The supernatants were collected for ELISA. Cells were used for RNA extraction and quantitative PCR.

**Measurement of MCP-1 in the cell supernatants:** A commercial enzyme linked-immuno-sorbent assay (ELISA) kits (R&D systems) was used to quantify MCP-1 levels in the cell free supernatants, according to the manufacturer's protocols. The absorbance at 450 nm and 570 nm (reference wavelength) were read using a microplate reader (Synergy 2, Biotek). All experiments were performed in triplicate.

**Real-time reverse transcription-PCR analysis:** Total RNA was extracted using the RNeasy Kit (Qiagen, Valencia, CA) according to the manufacturer's instructions. After reverse transcription (RT) using the RNA PCR kit (AMV) version 2.1, PCR was performed using an ABI Prism 7500 Sequence Detection System (Applied Biosystems, Foster City, CA) and QuantiTect SYBR Green PCR Master Mix (Qiagen, Valencia, CA). All reactions were carried out in triplicate, and the mRNA copy numbers of specific genes among the total RNA extracts were calculated. All data were expressed as the mean +/- standard deviation of the triplicate wells.

### Results

Different amounts of cerivastatin can be loaded into the ECM patch to achieve the desired level of release for efficacy. When the patch was incubated with 100 µg/ml cerivastatin, the drug release was 15-20 µg for the first 4 days and then slowly released over the next 10 days.

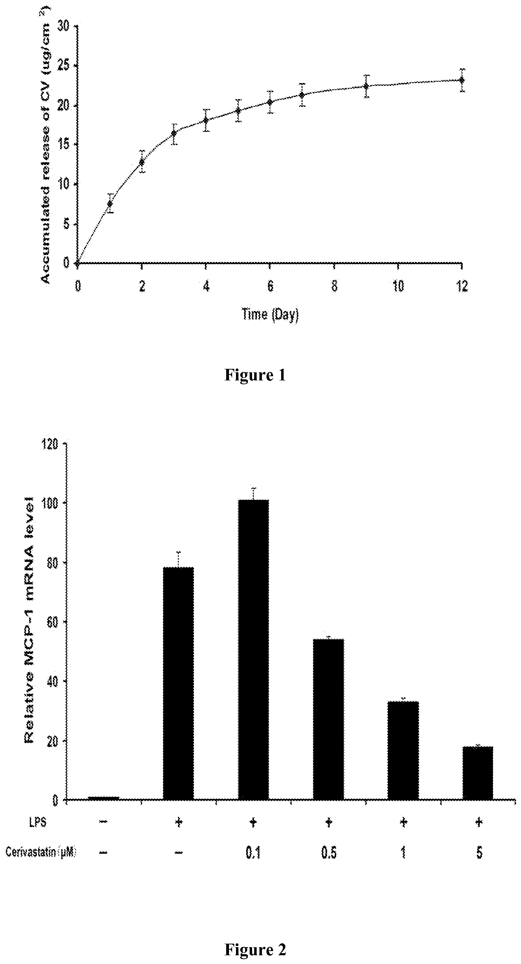

As shown in Figure 2, this level of cerivastatin was adequate to achieve complete inhibition of inflammatory chemokine MCP-1 generation by human monocyte cells THP-1. Much greater levels of cerivastatin (800-900 **µ**g/cm2) can be loaded into the patch when incubated at a higher concentration (2 mg/ml). Levels of loading and release are dependent on the statin that is used.

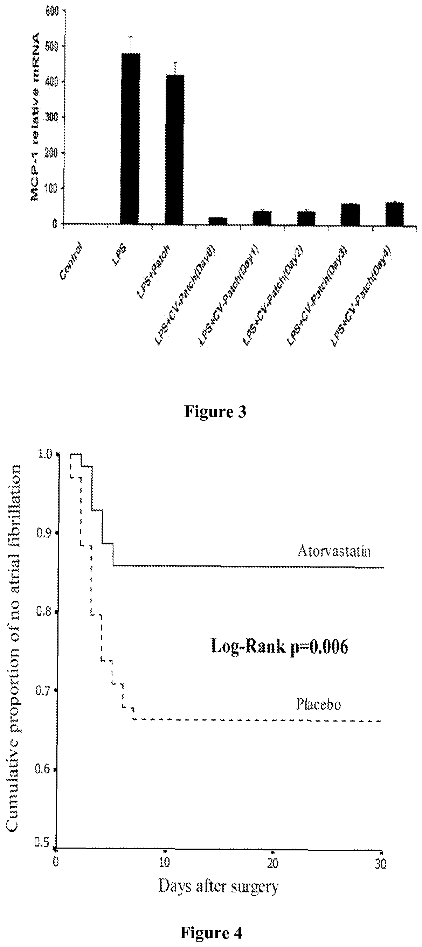

Figure 3 shows that when the patch was loaded by incubation with 100 **µ**g/ml cerivastatin and then placed on the upper chamber of a transwell plate in which inflammatory cells were placed in lower chamber such that the patch and the cells were separated by the culture medium, the cerivastatin released from the patch diffused to the cells and blocked the generation of proinflammatory chemokine MCP-1. These conditions are representative of *in vivo* conditions. The amount of cerivastatin released from the patch was sufficient to produce this effect for more than 4 days. These data show that cerivastatin amounts would be sufficient for blocking inflammation mediator during AF which mostly occurs during the 2-4 days post cardiac surgery.

Cerivastatin also blocked expression of the inflammatory receptor CCR2. This activity of cerivastain was different from other statins and thus may produce a greater impact in decreasing inflammation after surgery

Figure 4 shows that patients pretreated with atorvastatin orally had a reduced incidence of AF. These data suggest that local delivery of low dose statin, at a does expected to produce efficacy in treatment of AF, would not have the risk associated with systemically delivered statin. Furthermore, local delivery using the patch can achieve desired and consistent level in the target tissue.

### Example 2. Determination of the in vivo efficacy of the cerivastatin eluting tissue engineered patch in the dog model of AF.

The release kinetics demonstrated in Example 1 is suitable to produce tissue levels of cerivastatin to produce biological efficacy of cerivastatin for about a week. Since the majority of AF occurs 2-3 days post coronary artery bypass graft ("CABG"), the patch should provide ideal local drug treatment. The dog model of pericardites has been previously used for testing efficacy of systemically delivered statin. See, for example, Nattell, et al. Prog Biophys Mol Biol. 98(2-3):328-39 (2008); Okumura et al., J Am Coll Cardiol. 17(2):509-18 (1991); Pagé et al. J Am Coll Cardiol. 8(4):872-9 (1986).

## Claims

1. A polymeric matrix comprising a HMG CoA reductase inhibitor for use in the treatment of atrial fibrillation by local application and delivery of the inhibitor to cardiac tissue.

2. The polymeric matrix of claim 1 wherein the HMG CoA reductase inhibitor is a statin selected from the group consisting of atorvastatin, fluvastatin, lovastatin, pitavastatin, pravastatin, rosuvastatin, and simvastatin, cerivastatin, and combinations thereof.

3. The polymeric matrix of claim 1 comprising a natural polymer, optionally selected from the group consisting of extracellular matrix material, alginate, collagen and fibrin.

4. The polymeric matrix of claim 1 comprising a biodegradable polymer in the form of a hydrogel, woven or non-woven matrix.

5. The polymeric matrix of claim 1 that is applied as a spray or as a biological glue that adheres to the target tissue and delivers the statin.

6. The polymeric matrix of claim 1 further comprising one or more other therapeutic or prophylactic agents.

7. The polymeric matrix of claim 1 in a form that can be sutured to tissue.

8. The polymeric matrix of claim 1 which is a coating or layer on another substrate.

9. The polymeric matrix of any one of claims 1-8 which is administered during surgery.

10. The polymeric matrix of any one of claims 1-8 which is administered through a catheter or as part of a stent.

## Patentansprüche

1. Polymermatrix umfassend einen HMG-CoA-Reduktaseinhibitor zur Verwendung in der Behandlung von Vorhofflimmern durch lokale Anwendung und Zuführen des Inhibitors zum Herzgewebe.

2. Polymermatrix nach Anspruch 1, wobei der HMG-CoA-Reduktaseinhibitor ein Statin ausgewählt aus der Gruppe bestehend aus Atorvastatin, Fluvastatin, Lovastatin, Pitavastatin, Pravastatin, Rosuvastatin und Simvastatin, Cerivastatin, und Kombinationen daraus ist.

3. Polymermatrix nach Anspruch 1, umfassend ein natürliches Polymer, optional ausgewählt aus der Gruppe bestehend aus extrazellulärem Matrixmaterial, Alginat, Kollagen und Fibrin.

4. Polymermatrix nach Anspruch 1, umfassend ein biologisch abbaubares Polymer in Form eines Hydrogels, einer gewebten oder nicht-gewebten Matrix.

5. Polymermatrix nach Anspruch 1, die als Spray oder biologischer Kleber angewendet wird, die an das Zielgewebe anheftet und das Statin zuführt.

6. Polymermatrix nach Anspruch 1, die zusätzlich eine oder mehrere andere therapeutische oder prophylaktische Wirkstoffe umfasst.

7. Polymermatrix nach Anspruch 1, in einer Form, in der sie mit Gewebe vernäht werden kann.

8. Polymermatrix nach Anspruch 1, die eine Beschichtung oder eine Lage auf einem anderen Substrat ist.

9. Polymermatrix nach einem der Ansprüche 1 bis 8, die während eines chirurgischen Eingriffes verabreicht wird.

10. Polymermatrix nach einem der Ansprüche 1 bis 8, die über einen Katheter oder als Teil eines Stents verabreicht wird.

## Revendications

1. Matrice polymérique comprenant un inhibiteur de la HMG CoA réductase pour utilisation dans le traitement d'une fibrillation auriculaire par application locale et distribution de l'inhibiteur à un tissu cardiaque.

2. Matrice polymérique selon la revendication 1, dans laquelle l'inhibiteur de la HMG CoA réductase est une statine sélectionnée dans le groupe constitué par la atorvastatine, fluvastatine, lovastatine, pitavastatine, pravastatine, rosuvastatine et simvastatine, cérivastatine et leurs combinaisons.

3. Matrice polymérique selon la revendication 1, comprenant un polymère naturel, sélectionné facultativement dans le groupe constitué par un matériau de matrice extracellulaire, un alginate, un collagène et une fibrine.

4. Matrice polymérique selon la revendication 1, comprenant un polymère biodégradable sous la forme d'un hydrogel, d'une matrice tissée ou non tissée.

5. Matrice polymérique selon la revendication 1, qui est appliquée sous la forme d'une pulvérisation ou d'une colle biologique qui adhère au tissu cible et délivre la statine.

6. Matrice polymérique selon la revendication 1, comprenant en outre un ou plusieurs autres agents thérapeutiques ou prophylactiques.

7. Matrice polymérique selon la revendication 1, sous une forme qui peut être suturée au tissu.

8. Matrice polymérique selon la revendication 1, qui est un revêtement ou une couche sur un autre substrat.

9. Matrice polymérique selon l'une quelconque des revendications 1 à 8, qui est administrée pendant une intervention chirurgicale.

10. Matrice polymérique selon l'une quelconque des revendications 1 à 8, qui est administrée à travers un cathéter ou en tant que partie d'un stent.
